# EUROPEAN PATENT SPECIFICATION

(11) **EP 3 903 709 B1**
(45) Date of publication and mention of the grant of the patent: **09.10.2024**
(21) Application number: 19905526.0
(22) Date of filing: 27.12.2019
(51) Int. Cl.: A61B 18/14, A61B 18/08

(54) **SURGICAL TOOL**
CHIRURGISCHES INSTRUMENT
INSTRUMENT CHIRURGICAL

(30) Priority: 27.12.2018 CN 201811612725
(43) Date of publication of application: 03.11.2021
(73) Proprietor: Beijing Surgerii Robotics Company Limited, Beijing 100192 (CN)
(72) Inventor: XU, Kai, Beijing 100192 (CN); REN, Yitang, Beijing 100192 (CN)
(74) Representative: karo IP
(86) International application number: PCT/CN2019/128984
(87) International publication number: WO 2020/135664

(56) References cited:
- WO-A1-97/12557
- CN-A- 101 273 914
- CN-A- 107 106 179
- CN-A- 109 498 149
- CN-U- 209 611 301
- US-A1- 2003 191 494
- US-A1- 2004 193 146
- US-A1- 2005 004 432
- US-A1- 2007 244 508
- US-A1- 2018 193 086
- US-B1- 6 514 197
- US-B1- 6 514 197
- US-B1- 6 514 197
- US-B2- 7 354 439
- US-B2- 7 354 439

## Description

### Technical Field

The present disclosure relates to the field of medical instrument, in particular to a surgical tool.

### Background

Surgical forceps are commonly used for more delicate operations such as brain surgery, microsurgery, ENT (ear, nose and throat), obstetrics and gynecology, and hand surgery, and their use is gradually expanding. The prior art surgical forceps include a surgical tool head, a rod in the middle, and an actuator. The surgical tool head and the actuator are connected by the rod in the middle. The tail device in the prior art has a rigid structure and cannot be bent freely. Therefore, it cannot be integrated into a flexible surgical tool, which limits its use range. The document WO9712557A1 relates to the field of elongated, flexible forceps devices for grasping, culling, removing or otherwise manipulating tissues and other materials inside a patient. The document US2004193146A1 implements a robotically controlled medical instrument for grasping an item with a tool used to perform a medical procedure on a subject, as well as methods of using the instrument for such procedures. The document US6514197B1 relates to a treatment tool, which is inserted through a treatment tool insert channel of an endoscope and used for an operation within a body. The document US7354439B2 relates to a treatment tool for an endoscope, which is provided with an end effector including a pair of manipulation members pivotably supported at the distal end of the treatment tool so as to operate like pincers. US 2018/193086 A1 discloses the preamble of claim 1.

Therefore, there is a need in the art for a surgical tool that can be flexible.

### Summary

The invention is defined in claim 1.

An object of the present disclosure is to provide a surgical tool, comprising: a surgical tool head and a shaft, wherein the surgical tool head is connected with the shaft, and the shaft is a flexible shaft and comprises: a flexible core; a flexible sleeve, the flexible sleeve having an inner cavity, and the flexible core being slidably disposed in the inner cavity; wherein the surgical tool head comprises two jaws, the flexible core and the flexible sleeve are respectively connected with the two jaws, and the flexible core is able to slide relatively to the flexible sleeve to drive the two jaws to make relative opening and closing movements.

In a preferable embodiment, the flexible core comprises: an elastic metal wire; a first covering tube wrapping the elastic metal wire and fixed relatively to the elastic metal wire.

In a preferable embodiment, the flexible sleeve comprises: a flexible metal tube forming the inner cavity; and a second covering tube wrapping the metal tube and fixed relatively to the metal tube.

In a preferable embodiment, the two jaws are two electrodes, and the two electrodes are connected with the metal tube and the elastic metal wire, respectively.

In a preferable embodiment, the metal tube is formed of a spiral metal band, a metal wire mesh or a corrugated metal tube.

In a preferable embodiment, the elastic metal wire is made of nickel-titanium alloy.

In a preferable embodiment, the first covering tube is made of wear-resistant resin.

In a preferable embodiment, the second covering tube is made of resin or heat shrinkable tube.

In a preferable embodiment, the first covering tube is made of polytetrafluoroethylene.

In a preferable embodiment, the flexible sleeve extends to inside of the surgical tool head.

Compared with the prior art, the shaft of the surgical tool according to the present disclosure can be freely bent to any shape, and thus can be integrated into a flexible surgical tool and applied to a wider range during surgery.

### Brief Description of the Drawings

FIG. 1 is a schematic diagram of an embodiment of a surgical tool according to the present disclosure.
FIG. 2 is a perspective view of an embodiment of a surgical tool according to the present disclosure.
FIGure 3 is a half-cut perspective view of a surgical tool according to the present disclosure.
FIG. 4 is a schematic diagram of another embodiment of a surgical tool according to the present disclosure.
FIG. 5 is a perspective view of another embodiment of a surgical tool according to the present disclosure.
FIG. 6 is an internal structure diagram of a surgical tool head according to another embodiment of the surgical tool of the present disclosure.

### Detailed Description of Embodiments

Hereinafter, the preferred embodiments of the present disclosure will be described in detail with reference to the accompanying drawings, so as to better understand objects, features and advantages of the present disclosure.

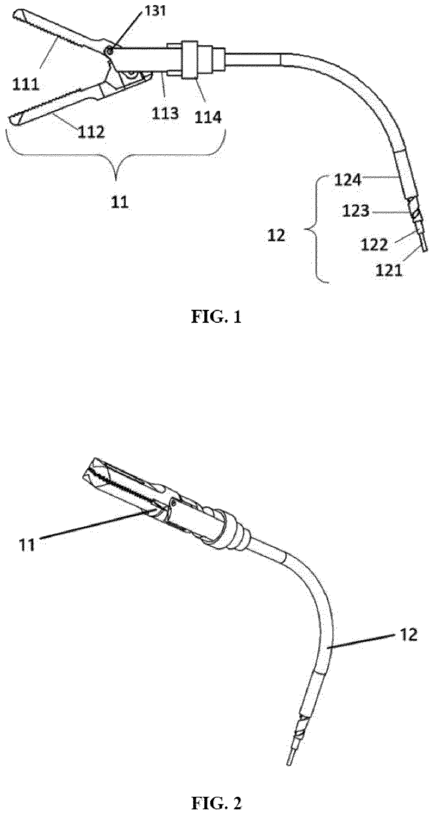

FIG. 1 is a schematic diagram of a surgical tool head 11 and a shaft 12 of an embodiment of a surgical tool according to the present disclosure. The shaft 12 is a flexible rod and can be bent freely. The surgical tool head 11 includes a first jaw 111, a second jaw 112, a base 113 and an insulator 114. The shaft 12 has a multi-layer nested structure. The shaft 12 has a flexible core. In the illustrated embodiment, the flexible core is a superelastic metal wire 121, and a first covering tube 122 is wrapped around the superelastic metal wire 121. The first covering tube 122 tightly wraps the superelastic metal wire 121 and is fixed relatively to the superelastic metal wire 121. However, it can be understood that the flexible core of the present disclosure is not limited to this, and any core that is flexible and can be bent falls within the scope of the present disclosure. A sleeve is provided outside a first covering tube 122. The sleeve has an inner cavity. The sleeve can include a metal tube 123 and a second covering tube 124, and the superelastic metal wire 121 and the first covering tube 122 can be slidably disposed in an inner cavity of the metal tube 123. In the illustrated embodiment, the metal tube 123 forms an inner cavity, and the superelastic metal wire 121 and the first covering tube 122 are slidably disposed in the inner cavity of the metal tube 123. Second covering tube 124 is wrapped on the outside of the metal tube 123. Similarly, the second covering tube 124 tightly wraps the metal tube 123 and is fixed relatively to the metal tube 123, thereby isolating the metal tube 123 from the outside. In such a structure, the superelastic metal wire 121 and the metal tube 123 are respectively connected with two jaws 111 and 112 of the surgical tool head 11. The superelastic metal wire 121 and the first covering tube 122 can slide reciprocatingly in the inner cavity of the metal tube 123 with respect to the metal tube 123 and the second covering tube 124. The reciprocating sliding can make the two jaws of the tool head 11 open and close. The movement of the superelastic metal wire 121 and the first covering tube 122 relative to the metal tube 123 and the second covering tube 124 can be realized by an actuator (not shown) connected with the shaft 12. The actuator may be in various forms, such as a handle of a scissor structure or any other device that enables the flexible core to slide relative to the flexible sleeve.

In the embodiment shown in FIG. 1, the two forceps 111 and 112 may be respectively formed as having electrodes, and the two electrodes are respectively electrically connected with the above-mentioned superelastic metal wire 121 and the metal tube 123. Therefore, the two electrodes can be supplied with power through the superelastic metal wire 121 and the metal tube 123. As such, the surgical tool can be used as a bipolar coagulation forceps to achieve blood coagulation and other functions. The first covering tube 122 and the second covering tube 124 can be used as insulation layers to electrically insulate the superelastic metal wire 121 and the metal tube 123 and the metal tube 123 from the outside, respectively.

The metal tube 123 also has a supporting function, which can keep the inner cavity from collapsing and maintain the gap between the metal tube 123 and the first covering tube 122, so as to prevent reciprocating sliding of the superelastic metal wire 121 and the first covering tube 122 from pulling the metal tube 123, especially in the case that the metal tube 123 is made of spiral metal band and metal wire mesh. The metal tube 123 may be in various forms as long as the metal tube 123 can be easily bent. For example, as shown in FIG. 1, the metal tube 123 is in a form of a spiral metal band. The metal tube 123 may also be formed of a metal wire mesh or a corrugated metal tube.

Preferably, a gap is formed between the first covering tube 122 and the metal tube 123, and the gap is preferably uniform along a length of the shaft 12 to prevent movement resistance of the superelastic metal wire 121 and the first covering tube 122 from increasing due to compression from the outside. In addition, the inner cavity of the metal tube 123 extends to the inside of the surgical tool head 11. Since the surgical tool is a medical instrument, it has a strict sterilization procedure. Therefore, the surgical tool can be sterilized more effectively by injecting water and disinfectant into the inner cavity.

The superelastic metal wire 121 may be made of any suitable metal. Preferably, the superelastic metal wire 121 is made of nickel-titanium alloy, which can be bent in any direction, and the nickel-titanium alloy has good memory ability, which facilitates the restoration of the shaft 12 to its original shape. The first covering tube 122 is usually made of a wear-resistant resin to withstand the reciprocating movement relative to the metal tube 123, thereby extending the life of the surgical tool. Preferably, the first covering tube 122 is made of polytetrafluoroethylene. A smooth surface of a polytetrafluoroethylene tube and its non-stick type can reduce the resistance of liquid flow, reduce the attachment of bacterium plaques, and improve the disinfection effect.

The second covering tube 124 is preferably made of resin. To facilitate manufacturing, a heat shrinkable tube may also be used to form the second covering tube 124.

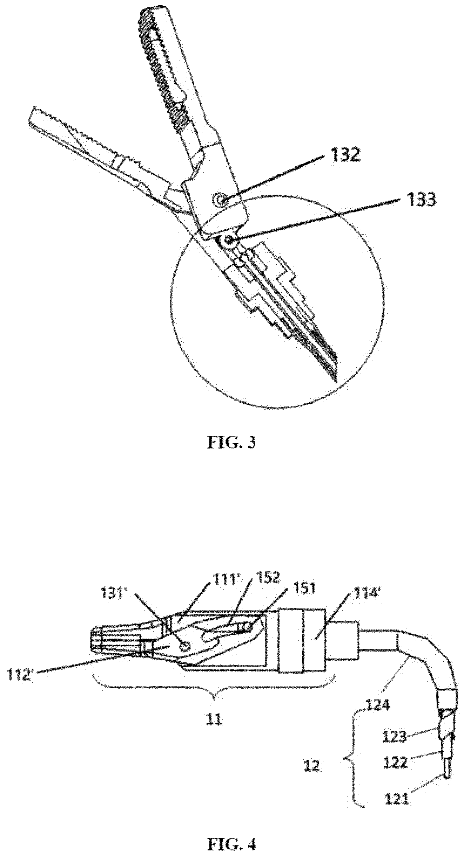

In the embodiment shown in FIG. 1, the surgical tool head 11 includes an insulator 114, a base 113 and two jaws 111 and 112. The shaft 12 is connected with the two electrodes and passes through the insulator 114. The base 113 extends from the insulator 114 toward the jaws. In the illustrated embodiment, the base 113 includes two extension parts extending from opposite positions at outer periphery of the insulator 114. The first jaw 111 is pivotally connected with the base 113 by a pivot 131, and as shown in FIG. 3, the second jaw 112 is pivotally connected with the jaw 111 by a pivot 132 at a position closer to the insulator 114 than the pivot 131. The second jaw 112 is pivotally connected with the superelastic metal wire 121 at an end close to the insulator 114 by a pivot 133. Therefore, when the superelastic metal wire 121 and the first insulating tube 122 reciprocatingly slide in the metal tube 123, the second jaw 112 is driven to pivot around the pivot 132 relatively to the first jaw 111, and the first jaw 111 is driven to pivot around the pivot 131, thereby realizing opening and closing of the two jaws 111 and 112. In addition, in the case where the two jaws 111 and 112 are used as electrodes, an insulating sleeve may be provided on outer surface of a pivot pin at the pivot 132 to ensure insulation between the two electrodes.

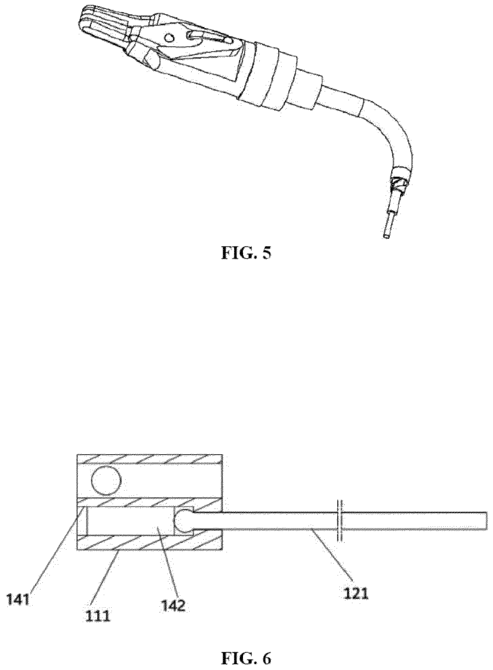

FIG. 4 and 5 respectively show a schematic diagram and a perspective view of a surgical tool head 11 and a shaft 12 of a surgical tool according to a second embodiment of the present disclosure. The structure of the shaft 12 is the same as the above-mentioned embodiment. The surgical tool head 11 includes an insulator 114', two jaws 111' and 112'. The first jaw 111' is fixed to the insulator 114', and the second jaw 112' has a "Y" shape and is pivotally connected with the first jaw 111' at a pivot 131'. The pivot 131' and the insulators 114' are separated by a certain distance. As shown in FIG. 6, an axial sliding channel 141 is provided in the first jaw 111', and the superelastic metal wire 121 is connected with the slider 142 in the sliding channel, so that sliding of the superelastic metal wire 121 and the first insulating tube 122 in the metal tube 123 drives the slider 142 to slide in the sliding channel 141. In addition, protrusions 151 are provided on both sides of the slider 142. The jaw 112' has two branch parts close to the insulator 114'. The two branch parts are provided with arc-shaped sliding grooves 152. The protrusions 151 can slide in the sliding grooves 152. Therefore, sliding of the slider 142 and the protrusions 151 through the sliding grooves 152 drives the second jaw 112' to pivot relatively to the first jaw 111', thereby achieving opening and closing between the two jaws. In addition, in the case where the two jaws 111 and 112 are used as electrodes, an insulating sleeve may be provided on outer surface of a pivot pin at 131' to ensure insulation between the two electrodes.

## Claims

1. A surgical tool, comprising:
a surgical tool head (11) comprising a first jaw (111, 111') and a second jaw (112, 112'); and
a flexible shaft (12) connected with the surgical tool head (11) and comprising:
a flexible core connected with the first jaw (111, 111'); and
a flexible sleeve connected with the second jaw (112, 112'), the flexible sleeve comprising an inner cavity, and the flexible core being slidably disposed in the inner cavity and operable to slide relatively to the flexible sleeve to drive relative opening and closing of the first jaw (111, 111') and the second jaw (112, 112');
wherein the flexible core comprises:
an elastic metal wire (121); a first covering tube (122) wrapping the elastic metal wire (121);
wherein the flexible sleeve comprises:
a metal tube (123) forming the inner cavity; a second covering tube (124) wrapping the metal tube (123) and fixed relatively to the metal tube (123);
wherein the first jaw (111, 111') comprises an electrode connected with the elastic metal wire (121), and the second jaw (112, 112') comprises an electrode connected with the metal tube (123),
**characterised in that**
the first covering tube (122) is fixed relatively to the elastic metal wire (121).

2. 4-The surgical tool according to claim 1, wherein the metal tube (123) comprises a spiral metal band, a metal wire mesh or a corrugated metal tube.

3. The surgical tool according to claim 1, wherein the elastic metal wire (121) comprises of nickel-titanium alloy.

4. The surgical tool according to claim 1, wherein the first covering tube (122) comprises wear-resistant resin.

5. The surgical tool according to claim 1, wherein the second covering tube (124) comprises resin or heat shrinkable tube.

6. The surgical tool according to claim 1, wherein the first covering tube (122) comprises polytetrafluoroethylene.

7. The surgical tool according to anyone of claims 1-6, wherein the flexible sleeve extends to inside of the surgical tool head (11).

8. The surgical tool according to any of claims 1-7, further comprising a base (113), wherein the first jaw (111, 111') is pivotally connected with the base (113) by a first pivot (131, 131') and pivotally connected with the second jaw (112) by a second pivot (132), and the first jaw (111) is pivotally connected with the flexible core by a third pivot (133).

9. The surgical tool according to claim 8, further comprising an insulating sleeve on outer surface of the second pivot (132).

10. The surgical tool according to any one of claims 1-7, further comprising:
a slider (142) connected with the flexible core and slidable in an axial sliding channel on the first jaw (111, 111').

11. The surgical tool according to claim 10, wherein the second jaw (112') comprises a first branch part having a first arc-shaped sliding groove (152) and a second branch part having a second arc-shaped sliding groove, and
the slider (142) comprises a first protrusion (151) slidable in the first arc-shaped sliding groove (152) and a second protrusion slidable in the second arc-shaped sliding groove.

## Patentansprüche

1. Ein chirurgisches Instrument, umfassend
einen chirurgischen Instrumentenkopf (11), umfassend eine erste Klemme (111, 111') und eine zweite Klemme (112, 112'); und
einen flexiblen Schaft (12), der mit dem chirurgischen Instrumentenkopf (11) verbunden ist und umfassend:
einen flexiblen Kern verbunden mit der ersten Klemme (111, 111'); und eine flexible Hülse verbunden mit einer zweiten Klemme (112, 112'), wobei die flexible Hülse einen inneren Hohlraum umfasst, und der flexible Kern gleitend in dem inneren Hohlraum angeordnet ist und relativ zu der flexiblen Hülse gleiten kann, um ein relatives Öffnen und Schließen der ersten Klemme (111, 111') und der zweiten Klemme (112, 112') zu bewirken;
wobei der flexible Kern Folgendes umfasst:
einen elastischen Metalldraht (121);
ein erstes Umhüllungsrohr (122), das den elastischen Metalldraht (121) umhüllt;
wobei die flexible Hülse Folgendes umfasst:
ein Metallrohr (123), das den inneren Hohlraum bildet;
ein zweites Umhüllungsrohr (124), das das Metallrohr (123) umhüllt und relativ zu dem Metallrohr (123) fixiert ist;
wobei die erste Klemme (111, 111') eine Elektrode umfasst, die mit dem elastischen Metalldraht (121) verbunden ist, und die zweite Klemme (112, 112') eine Elektrode umfasst, die mit dem Metallrohr (123) verbunden ist, **dadurch gekennzeichnet, dass**
das erste Umhüllungsrohr (122) relativ zu dem elastischen Metalldraht (121) fixiert ist.

2. Das chirurgische Instrument nach Anspruch 1, wobei das Metallrohr (123) ein spiralförmiges Metallband, einen Metalldrahtgitter oder ein Metallwellrohr umfasst.

3. Das chirurgische Instrument nach Anspruch 1, wobei der elastische Metalldraht (121) eine Nickel-Titan-Legierung umfasst.

4. Das chirurgische Instrument nach Anspruch 1, wobei das erste Umhüllungsrohr (122) ein verschleißfestes Harz umfasst.

5. Das chirurgische Instrument nach Anspruch 1, wobei das zweite Umhüllungsrohr (124) ein Harz oder einen Wärmeschrumpfschlauch umfasst.

6. Das chirurgische Instrument nach Anspruch 1, wobei das erste Umhüllungsrohr (122) Polytetrafluorethylen umfasst.

7. Das chirurgische Instrument nach einem der Ansprüche 1 - 6, wobei die flexible Hülse sich in den chirurgischen Instrumentenkopf (11) hinein erstreckt.

8. Das chirurgische Instrument nach einem der Ansprüche 1 - 7, weiter umfassend eine Basis (113), wobei die erste Klemme (111, 111') schwenkbar mit der Basis (113) an einem ersten Drehpunkt (131, 131') verbunden ist und schwenkbar mit der zweiten Klemme (112) an einem zweiten Drehpunkt (132) verbunden ist, und wobei die erste Klemme (111) schwenkbar mit dem flexiblen Kern an einem dritten Drehpunkt (133) verbunden ist.

9. Das chirurgische Instrument nach Anspruch 8, weiter umfassend eine isolierende Hülse auf einer Außenfläche des zweiten Drehpunkts (132).

10. Das chirurgische Instrument nach einem der Ansprüche 1 - 7, weiter umfassend: einen Gleiter (142), der mit dem flexiblen Kern verbunden ist und gleitbar in einem axialem Gleitkanal auf der ersten Klemme (111, 111') ist.

11. Das chirurgische Instrument nach Anspruch 10, wobei die zweite Klemme (112') ein erstes Abzweigteil mit einer ersten bogenförmigen Gleitrinne (152) und ein zweites Abzweigteil mit einer zweiten bogenförmigen Gleitrinne aufweist, und der Gleiter (142) einen ersten Vorsprung (151), der in der ersten bogenförmigen Gleitrinne (152) gleitbar ist, und einen zweiten Vorsprung, der in der zweiten bogenförmigen Gleitrinne gleitbar ist, umfasst.

## Revendications

1. Instrument chirurgical, comprenant :
une tête d'instrument chirurgical (11) comprenant une première mâchoire (111, 111') et
une seconde mâchoire (112, 112') ; et
une tige flexible (12) reliée à la tête d'instrument chirurgical (11) et comprenant :
un noyau flexible relié à la première mâchoire (111, 111') ; et
un manchon flexible relié à la seconde mâchoire (112, 112'), le manchon flexible comprenant une cavité interne, et le noyau flexible étant disposé de manière coulissante dans la cavité interne et apte à coulisser par rapport au manchon flexible pour entraîner une ouverture et une fermeture relatives de la première mâchoire (111, 111') et de la seconde mâchoire (112, 112') ;
dans lequel le noyau flexible comprend :
un fil métallique élastique (121) ;
un premier tube de couverture (122) enveloppant le fil métallique élastique (121) ;
dans lequel le manchon flexible comprend :
un tube métallique (123) formant la cavité interne ;
un second tube de couverture (124) enveloppant le tube métallique (123) et fixé par rapport au tube métallique (123) ;
dans lequel la première mâchoire (111, 111') comprend une électrode connectée au fil métallique élastique (121), et la seconde mâchoire (112, 112') comprend une électrode connectée au tube métallique (123),
**caractérisé en ce que**
le premier tube de couverture (122) est fixé par rapport au fil métallique élastique (121).

2. Instrument chirurgical selon la revendication 1, dans lequel le tube métallique (123) comprend une bande métallique en spirale, un maillage de fils métalliques ou un tube métallique ondulé.

3. Instrument chirurgical selon la revendication 1, dans lequel le fil métallique élastique (121) comprend un alliage nickel-titane.

4. Instrument chirurgical selon la revendication 1, dans lequel le premier tube de couverture (122) comprend une résine résistante à l'usure.

5. Instrument chirurgical selon la revendication 1, dans lequel le second tube de couverture (124) comprend une résine ou un tube thermorétractable.

6. Instrument chirurgical selon la revendication 1, dans lequel le premier tube de couverture (122) comprend du polytétrafluoroéthylène.

7. Instrument chirurgical selon l'une quelconque des revendications 1 à 6, dans lequel le manchon flexible s'étend vers l'intérieur de la tête d'instrument chirurgical (11).

8. Instrument chirurgical selon l'une quelconque des revendications 1 à 7, comprenant en outre une base (113), dans lequel la première mâchoire (111, 111') est reliée de manière pivotante à la base (113) par un premier pivot (131, 131') et reliée de manière pivotante à la seconde mâchoire (112) par un deuxième pivot (132), et la première mâchoire (111) est reliée de manière pivotante au noyau flexible par un troisième pivot (133).

9. Instrument chirurgical selon la revendication 8, comprenant en outre un manchon isolant sur une surface externe du deuxième pivot (132).

10. Instrument chirurgical selon l'une quelconque des revendications 1 à 7, comprenant en outre :
un coulisseau (142) relié au noyau flexible et pouvant coulisser dans un canal de coulissement axial sur la première mâchoire (111, 111').

11. Instrument chirurgical selon la revendication 10, dans lequel la seconde mâchoire (112') comprend une première partie de branche ayant une première rainure de coulissement en forme d'arc (152) et une seconde partie de branche ayant une seconde rainure de coulissement en forme d'arc, et
le coulisseau (142) comprend une première saillie (151) pouvant coulisser dans la première rainure de coulissement en forme d'arc (152) et une seconde saillie pouvant coulisser dans la seconde rainure de coulissement en forme d'arc.
